(19) Europäisches Patentamt
European Patent Office
Office européen des brevets

(11) **EP 4 239 328 B1**

(12) **EUROPEAN PATENT SPECIFICATION**

(45) Date of publication and mention
of the grant of the patent:
**01.04.2026 Bulletin 2026/14**

(21) Application number: **23155041.9**

(22) Date of filing: **06.02.2023**

(51) International Patent Classification (IPC):
**G01N 27/327** (2006.01) **G01N 33/487** (2006.01)

(52) Cooperative Patent Classification (CPC):
**G01N 27/3272; G01N 27/3274; G01N 33/48771**

(54) **TESTING IMPLEMENT AND MEASURING DEVICE**

TESTGERÄT UND MESSVORRICHTUNG

OUTIL DE TEST ET DISPOSITIF DE MESURE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **09.02.2022 JP 2022018765**

(43) Date of publication of application:
**06.09.2023 Bulletin 2023/36**

(73) Proprietor: **ARKRAY, Inc.**
**Kyoto-shi, Kyoto 601-8045 (JP)**

(72) Inventor: **Nishiyama, Hisashi**
**Kyoto-shi, Kyoto, 602-0008 (JP)**

(74) Representative: **Hoffmann Eitle**
**Patent- und Rechtsanwälte PartmbB**
**Arabellastraße 30**
**81925 München (DE)**

(56) References cited:
**EP-A1- 3 163 300     WO-A1-2009/031761
WO-A1-2017/039976  US-A1- 2009 030 617
US-B2- 7 955 856**

**Description**

BACKGROUND

Technical Field

The present disclosure relates to a testing implement and a measuring device.

Related Art

Japanese Patent No. 4885508, International Patent Application Publication No. 2003/029804, and Japanese Patent No. 3859239 disclose a technique in which, in order for a measuring device to be able to judge information (calibration curve information) of sensor sensitivity (sensitivity of a reagent) of a biosensor, information of measured items of the sensor, or information (incompatibility information) for judging whether or not the sensor is a sensor that corresponds to the connected measuring device, the measuring device uses, as an indicator of various information, the shape of a conductive material at the connection terminal portion (computing information indicating region) of the biosensor that is connected to the measuring device, or the electrical connection path when the biosensor is connected to the measuring device.

Japanese Patent No. 4885508, International Patent Application Publication No. 2003/029804, and Japanese Patent No. 3859239 disclose using the resistance value of an electrical connection path when connected to a measuring device, and devise the connecting of a conductive material. Japanese Patent No. 4885508 discloses a technique of judging the connection on the basis of the resistance value. Further, International Patent Application Publication No. 2003/029804 and Japanese Patent No. 3859239 disclose specific methods of judging whether or not a path is connected, by the on/off state ("0" or "1") of energization.

In the above-described Patent Documents, the resistance value or the absence/presence of connection between two points at a conductive material of a connection terminal portion (computing information indicating region) of a biosensor is an indicator of respective computing information. However, methods in which the resistance value between two points is used as an indicator of various information are affected by the resistance value of the conductive material itself. Namely, even if there are conductive materials of the same shape, the resistance values between two points thereof differ if there are differences in the manufacturing of the conductive materials themselves, or if there is damage to the conductive materials. If the resistance values vary, the measuring device cannot accurately judge the computing information. Further, a method that uses the absence/presence of connection between two points as an indicator of respective information is limited to the two selections of whether or not the path is connected, and there is a small amount of information.

WO 2017/039976 Al describes a sensor including a non-conductive substrate and a circuit on the non-conductive substrate. The circuit includes a primary resistive element on the non-conductive substrate having a first end and a second end, wherein the primary resistive element has a predetermined configuration; a secondary resistive element on the nonconductive substrate having a plurality of taps connected to the primary resistive element at a plurality of predetermined connection points on the predetermined configuration, the plurality of predetermined connection points defining a plurality of unique resistive paths through at least a portion of the predetermined configuration; and the plurality of unique resistive paths having a plurality of resistance values, the plurality resistance values determined using a non-linear distribution function. A sensor is configured to perform at least one of quantitative and qualitative analysis of an analyte in a sample of fluid. WO 2009/031761 Al relates to a biosensor for selectively performing quantitative analysis on a specific substance contained in a biological sample and a readout meter using the same. The biosensor comprises an electrode for recording identification information of the biosensor thereon. The electrode has an electrode pattern and a plurality of contact points formed thereon according to the identification information, and the identification information is recorded on the electrode based on the ratio of resistances between the plurality of contact points. Various items of identification information such as in-i formation on recognition of electrical connection between the contact points of the biosensor and a readout meter, the kind of a target substance, measurement conditions, production information of the biosensor, analysis information of the target substance,

user information, available meter information and the like are automatically recognized by the readout meter through the electrodes. US 2009/0030617 A1 describes a test strip on which a first conductive region is divided from a second conductive region by a break formed through the underlying conductive pattern in the test strip. US 2009/0030617 A1 describes a biosensor strip having an encoding pattern that is divided into separate circuits.

SUMMARY

The present disclosure was made in consideration of the above-described points, and an object thereof is to provide a testing implement and a measuring device that are difficult to affect by the resistance value of a conductive material itself at the time of outputting information for computing.

The present invention is defined by the claims.

In accordance with the present disclosure, there can be provided a testing implement and a measuring device that are difficult to affect by the resistance value of a conductive material itself at the time of outputting information for computing.

BRIEF DESCRIPTION OF THE DRAWINGS

Exemplary embodiments of the present disclosure will be described in detail based on the following figures, wherein:

Fig. 1 is a perspective view illustrating the exterior of a measuring device relating to an embodiment of the technique of the disclosure;
Fig. 2 is a schematic drawing of a biosensor used at the measuring device of the embodiment;
Fig. 3 is a drawing illustrating, in an enlarged manner, a computing information detection region of a biosensor;
Fig. 4 is a drawing illustrating, in an enlarged manner, the computing information detection region of the biosensor;
Fig. 5 is a block drawing illustrating functions of the measuring device of the embodiment;
Fig. 6 is a drawing illustrating modified examples of the shape of a conductive region;
Fig. 7 is a drawing illustrating modified examples of the shape of a cutting line;
Fig. 8A is a drawing illustrating an example in which a portion of the conductive region is trimmed;
Fig. 8B is a drawing illustrating an example in which a portion of the conductive region is trimmed;
Fig. 8C is a drawing illustrating an example in which a portion of the conductive region is trimmed; and
Fig. 8D is a drawing illustrating an example in which a portion of the conductive region is trimmed.

DETAILED DESCRIPTION

An example of an embodiment of the present disclosure is described hereinafter with reference to the drawings. Note that structural elements and portions that are the same in the drawings are denoted by the same reference numerals. Further, dimensional proportions in the drawings are exaggerated for convenience of explanation, and there are cases in which they differ from actual proportions.

(1) Testing Implement

The testing implement relating to the present embodiment is a testing implement utilized in a state of attachment to a measuring device that performs computing using information values representing a measurement target component within a sample based on information for computing, the testing implement being configured to output the information for computing to the measuring device, and including: a component measurement region disposed on a substrate formed from a non-conductive material, for measuring the measurement target component; a conductive region insulated from the component measurement region formed of a conductive material and disposed on the substrate, and at which a voltage application point to which a predetermined voltage is applied, an earth connection point connected to ground potential, and at least one measurement point for measuring an electrical measurement value, are established; and a resistance prescription region that prescribes a ratio, within the conductive region, between a first resistance value at the conductive region between the voltage application point and the earth connection point, and a second resistance value at the conductive region between the voltage application point or the earth connection point, and the measurement point; wherein the resistance prescription region is a region within the conductive region;

wherein the resistance prescription region is a non-conductive region between the voltage application point and the earth connection point;
wherein the resistance prescription region is a non-conductive cutting line that incompletely surrounds a periphery of the measurement point.

At this testing implement, the ratio between a first resistance value and a second resistance value is prescribed, and an electrical measurement value is measured at a measurement point, and effects of the resistance value of the conductive material itself at the time when information for computing is outputted can thereby be suppressed.

The material is, for example, a bodily fluid or a liquid for water quality inspection. Examples of bodily fluids are blood, plasma, urine, saliva, and the like.

The measurement target component means a chemical component included in a sample. For example, in a case in which the sample is blood, the measurement target component is glucose (blood sugar) or lactate (lactic acid) or the like that is included in the blood.

The substrate is formed from a non-conductive material that is a material that is electrically non-conductive, and is formed from, for example, an arbitrary insulating material that cannot carry charges or current. Examples thereof are polyester, polyethylene, polyethylene terephthalate, polycarbonate, vinyl, and the like.

The component measurement region is the region that is disposed at the testing implement in order to measure the measurement target component, and is structured such that the measurement target component can be measured by electrochemical means or optical means for example. A reagent that reacts with the measurement target component is provided at the component measurement region as needed. In the case of an electrochemical means, the component measurement region is prepared such that the amount of the measurement target component can be measured by measuring the amount of electrons generated due to the reagent reacting with the measurement target component. Further, in the case of an optical means, the component measurement region is prepared such that the amount of the measurement target component can be measured by measuring the change in color tone that arises due to the reagent reacting with the measurement target component.

The conductive region is formed by a conductive material that is disposed on the substrate, by using a metal material such as gold (Au) or a carbon material such as carbon. A voltage application point, an earth connection point, and at least one measurement point are respectively established so as to be apart from one another at the conductive region on the surface that is exposed in the direction opposite the reverse surface of the substrate. These respective points are positioned on conductive materials, and can be electrically connected without being disconnected, and preferably are positioned on the same conductive material. The voltage application point is a point within the conductive region to which voltage is applied from the measuring device at the time when the testing implement is attached to the measuring device. The earth connection point is a point within the conductive region where the voltage is connected to earth (the voltage is zero) or to a predetermined potential (fixed potential). The measurement point is on the path on which current flows from the voltage application point to the earth connection point, and a point within the conductive region is established as the measurement point.

The resistance prescription region is a region within the conductive region, for prescribing the ratio between a first resistance value at the conductive region between the voltage application point and the earth connection point, and a second resistance value at the conductive region between the voltage application point or the earth connection point and the measurement point. The resistance prescription region is a structure for setting at least one of the first resistance value and the second resistance value to an arbitrary resistance value. The conductive region between the voltage application point and the earth connection point means a region within the conductive region where the testing implement is attached to the measuring device, and where a potential difference arises between the voltage application point and the earth connection point at the time when voltage is applied from the measuring device to the voltage application point, and can also be called a region within the conductive region where current flows. Similarly, the conductive region between the voltage application point or the earth connection point and the measurement point also means a region within the conductive region where a potential difference arises between the voltage application point or the earth connection point and the measurement point, and can also be called a region within the conductive region where current flows. Note that the conductive region between the voltage application point or the earth connection point and the measurement point is a portion of the conductive region between the voltage application point and the earth connection point.

The resistance value of the conductive region between the voltage application point and the earth connection point, and the resistance value of the conductive region between the voltage application point or the earth connection point and the measurement point, are prescribed as arbitrary resistance values, in order to prescribe at least one of the first resistance value and the second resistance value as an arbitrary resistance value. Namely, it can also be said the resistance value of at least one conductive region among the conductive region between the voltage application point and the measurement point and the conductive region between the earth connection point and the measurement point is prescribed as an arbitrary resistance value. As the means for prescribing the resistance value, the resistivity of the conductive material at that conductive region can be prescribed, or at least one of the length, width or thickness of a conductive region between two arbitrary points can be prescribed.

Specifically, in order to prescribe the ratio between the first resistance value and the second resistance value, for example, the distance between the voltage application point and the earth connection point, and the distance between the voltage application point or the earth connection point and the measurement point, at the resistance prescription region may be prescribed.

The resistance prescription region is a cutting line that surrounds the periphery of the measurement point and at which one portion is open. The ratio between the first resistance value and the second resistance value can be prescribed by providing such a cutting line.

The cutting line that surrounds the periphery of the measurement point may surround the periphery of the measurement point in the form of a rectangle. Due to the cutting line surrounding the periphery of the measurement point in a rectangular form, the distance between the voltage application point and the earth connection point can be made to be long.

The cutting line that surrounds the periphery of the measurement point may open at a position in a vicinity of the measurement point. Due to the cutting line being open at a position in a vicinity of the measurement point, the distance between the voltage application point or the earth connection point and the measurement point can be made to be short.

Further, for example, the ratio between the first resistance value and the second resistance value may be prescribed by providing, as the resistance prescription region, a non-conductive region between the voltage application point and the earth connection point.

(2) Measuring Device

The measuring device relating to the present embodiment is a measuring device utilizing the above-described testing implement by the testing implement being attached thereto, the measuring device carrying out computation using information values representing a component that is an object of measurement within a sample on the basis of the information for computing outputted from the testing implement, and including: a voltage applying terminal connected to the voltage application point in order to apply voltage to the conductive material; a ground terminal connected to the earth connection point in order to make the earth connection point be ground potential; a measurement terminal connected to the measurement point; an information measuring section that measures an electrical measurement value of the measurement point; and an information for computing specifying section that specifies information for computing on the basis of the electrical measurement value measured by the information measuring section.

The electrical measurement value of the measurement point is the potential, the current value, the resistance value or the like, and preferably is the potential of the measurement point.

The measuring device may further have a terminal for component measurement that is connected to the component measurement region of the testing implement, and a measuring section that measures the measurement target component by using the component measurement region.

In the following description, "upstream side" and "downstream side" are defined along the direction in which blood, which is applied in the form of a drop or drops on a biosensor, flows within a flow path.

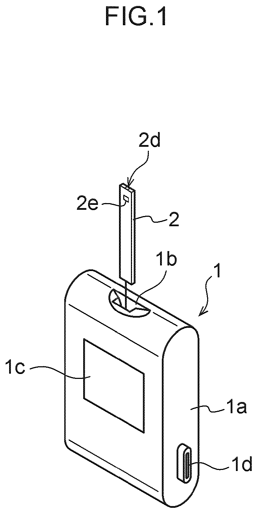

Fig. 1 is a perspective view illustrating the exterior of a measuring device 1 relating to the present embodiment. As an example, the present embodiment is an example of a case in which the measuring device 1 is a portable blood sugar value meter. In Fig. 1, a portable blood sugar value meter serving as the measuring device 1, and a biosensor 2 that is structured so as to be able to be attached to and removed from the measuring device 1, are provided. The biosensor 2 is an example of the testing implement of the present disclosure. A blood supply port 2d serving as an introduction port to a flow path 2a, which is described later, in order for the blood of a patient that serves as a sample to be introduced into the flow path 2a, and an air hole 2e for discharging air that is within the flow path 2a due to the introduction of the blood, are formed at the biosensor 2. The biosensor 2 functions to detect the blood sugar value (glucose value) within the blood. The measuring device 1 illustrated in Fig. 1 can be used as a blood sugar value meter such as, for example, a portable blood sugar measuring device, a blood sugar self-measuring meter, or the like.

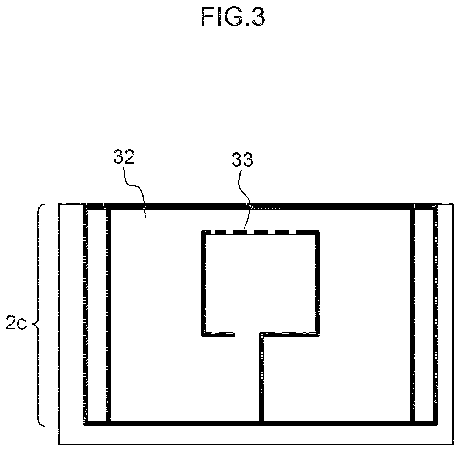

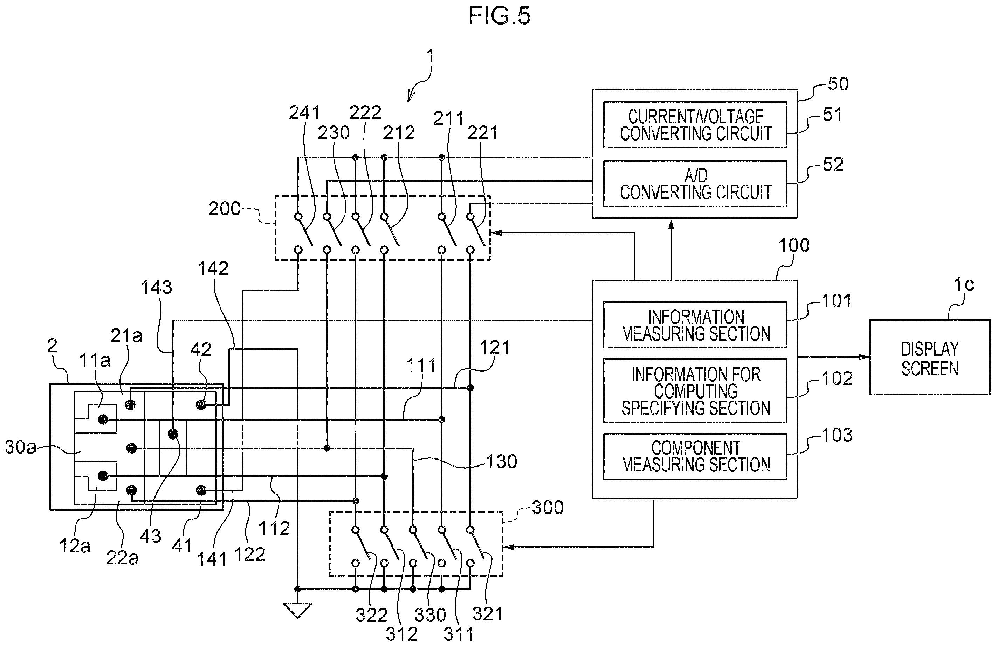

The measuring device 1 has a main body 1a, and an insertion port 1b for insertion of the strip-shaped biosensor 2 is provided in this main body 1a. Further, as illustrated in Fig. 3, a voltage applying device 50, which supplies a predetermined voltage signal to the biosensor 2 and receives a current signal expressing the results of measurement from the biosensor 2 and carries out A/D conversion, is provided at the main body 1a. Further, as illustrated in Fig. 5, a control section 100, which is structured by a microprocessor for example and which carries out control of the respective sections of the measuring device 1, is provided at the main body 1a. As illustrated in Fig. 5, the control section 100 causes a predetermined voltage signal to be supplied from the voltage applying device 50 to the biosensor 2, and generates measured data expressing a measurement value on the basis of the current value from the biosensor 2 that corresponds to the supply of the voltage signal. The measured data obtained at a measuring section is recorded in an unillustrated recording section. The measured data obtained at the control section 100 is recorded in the recording section in association with the time of measurement, the patient ID, or the like.

Further, a display screen 1c that displays the measured data, and a connector 1d for data communication with an external device, are provided at the main body 1a. The connector 1d transmits and receives data, such as the measured data, the measurement time, the patient ID and the like, between the measuring device 1 and a portable device such as a smartphone or the like, or a personal computer or the like, that serves as the external device. Namely, the measuring device 1 is structured such that, via the connector 1d, the measured data or the measurement time can be transferred to an external device, and the patient ID and the like can be received from an external device and associated with the measured data or the like.

Note that, other than the structure described above, for example, there may be a structure in which the control section 100 is provided at an end portion of the biosensor 2, and the measured data is generated at the biosensor 2 side. Further, a user interface including an input portion such as a button, a touch panel or the like for a user such as the patient or the like to input data, may be provided at the main body 1a of the measuring device 1. Moreover, the display screen 1c or the recording section or the like may be provided at an external device that can be connected to the main body 1a, and not provided at the main body 1a.

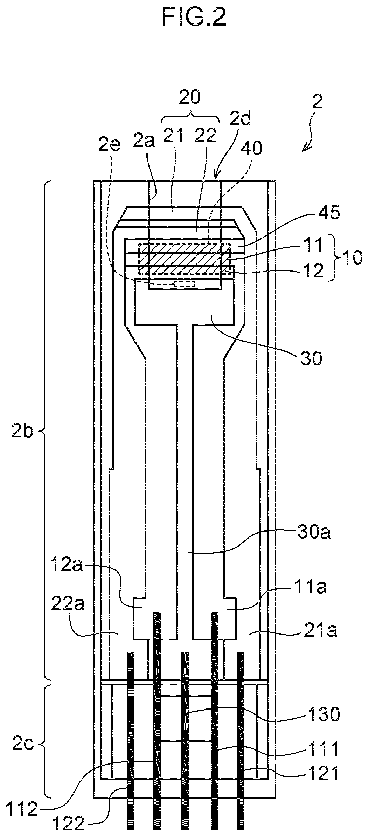

Fig. 2 is a schematic drawing of the biosensor 2 that is used at the measuring device 1 of the present embodiment. In the drawing, the upper side is the upstream side, and the lower side is the downstream side. At the biosensor 2, for example, an electrode layer, which is formed by using a metal material such as gold (Au), or a carbon material such as carbon, is formed on a substrate formed by using a synthetic resin (plastic). An unillustrated spacer having a rectangular cut-out portion serving as a covered region is layered on the electrode layer, and an unillustrated cover, which is formed of a synthetic resin and in which the air hole 2e is formed, is layered on the spacer. A space having the blood supply port 2d that is formed by the cut-out portion of the spacer is formed due to the layering of the substrate, the spacer and the cover, and this space is the flow path 2a. The air hole 2e is formed in a vicinity of the downstream end of the flow path 2a.

In the present embodiment, the electrode layer is formed such that five electrodes that are a first working electrode 11 and a first counter electrode 12 that serve as a first electrode pair 10, a second working electrode 21 and a second counter electrode 22 that serve as a second electrode pair 20, and a blood sensing electrode 30, are formed in parallel in the length direction and the width direction respectively of the biosensor 2 within the flow path 2a, so as to be exposed in rectangular shapes. The first electrode pair 10, the second electrode pair 20 and the blood sensing electrode 30 that are exposed at the flow path 2a contact the blood that is introduced-in, and function as the component measurement region. Note that regions between adjacent electrodes are insulated. For example, in a case in which the electrode layer is formed of a metal material that is formed by physical vapor deposition, the regions between the respective electrodes are insulated by drawing a predetermined electrode pattern by laser light (trimming). Further, in the case of an electrode layer is that formed by using a carbon material, the respective electrodes are formed with a predetermined interval therebetween. The electrode layer of the present embodiment is formed by using a nickel vanadium alloy. In the present embodiment, the first electrode pair 10 is an electrode pair for acquiring the glucose value, and the second electrode pair 20 is an electrode pair for acquiring the hematocrit value. The shapes of the first electrode pair 10, the second electrode pair 20 and the blood sensing electrode 30 are not limited to those illustrated in Fig. 2.

The respective electrodes extend along the length direction of the biosensor 2, and are bent in the width direction at the upstream end sides thereof. These bent portions are positioned parallel in the width direction in the order, from the upstream side, of the second working electrode 21, the second counter electrode 22, the first working electrode 11, the first counter electrode 12, and the blood sensing electrode 30. The respective electrodes are covered by the aforementioned unillustrated cover at a component measurement region 2b that is from the upstream end to a vicinity of the downstream end of the biosensor 2, but the downstream end portions are exposed without being covered, and this portion is the region that is inserted into the insertion port 1b of the main body 1a. At the downstream end portion of this component measurement region 2b, a lead portion 11a of the first working electrode 11, a lead portion 12a of the first counter electrode 12, a lead portion 21a of the second working electrode 21, a lead portion 22a of the second counter electrode 22, and a lead portion 30a of the blood sensing electrode 30 respectively are contacts that are exposed.

At the width direction central portion of the upstream portion of the biosensor 2, a gap is formed between the respective electrodes and the unillustrated cover. This gap is the capillary-shaped flow path 2a to which blood is applied as a drop or droplets and through which the blood flows as described above. Further, a non-conductive region 45, which is the gap between the second counter electrode 22 that is the second electrode when counted from the upstream side and the first working electrode 11 that is the third electrode, is wider than the gaps between the other electrodes. This non-conductive region 45 is a region that is formed to be insulated from the other electrodes, due to a rectangular pattern being drawn on the electrode layer by laser light. Moreover, a reagent 40 is placed on the first working electrode 11. The reagent 40 is a chemical substance that reacts with the object of measurement, and, for example, may be a substance that includes an enzyme and a mediator. The downstream side of the region where the reagent 40 is placed extends to the middle of the first counter electrode 12, and the upstream side extends to the middle of the non-conductive region 45 but does not reach the second counter electrode 22. In other words, because the first working electrode 11 and the second counter electrode 22 are separated by the non-conductive region 45, contact between the second counter electrode 22 and the reagent 40 that is placed on the first working electrode 11 is impeded. When blood is applied in a drop or drops to the blood supply port 2d of the biosensor 2, the blood flows within the flow path 2a due to capillary action toward the downstream side in the order of the second counter electrode 22, the first working electrode 11, the first counter electrode 12 and the blood sensing electrode 30. At this time, when the blood reaches the first working electrode 11, the reagent 40 placed on the first working electrode 11 is dissolved by the blood.

Terminals 111, 112, 121, 122, 130 for component measurement, which are provided at the measuring device 1 and contact the respective lead portions of the component measurement region 2b due to the insertion of the biosensor 2, are illustrated in Fig. 2. The terminal 111 for component measurement contacts the lead portion 11a of the first working electrode 11. The terminal 112 for component measurement contacts the lead portion 12a of the first counter electrode 12. The terminal 121 for component measurement contacts the lead portion 21a of the second working electrode 21. The terminal 122 for component measurement contacts the lead portion 22a of the second counter electrode 22. The terminal 130 for component measurement contacts the lead portion 30a of the blood sensing electrode 30. Note that the terminals 111, 112, 121, 122, 130 for component measurement point-contact the respective lead portions due to the insertion of the biosensor 2.

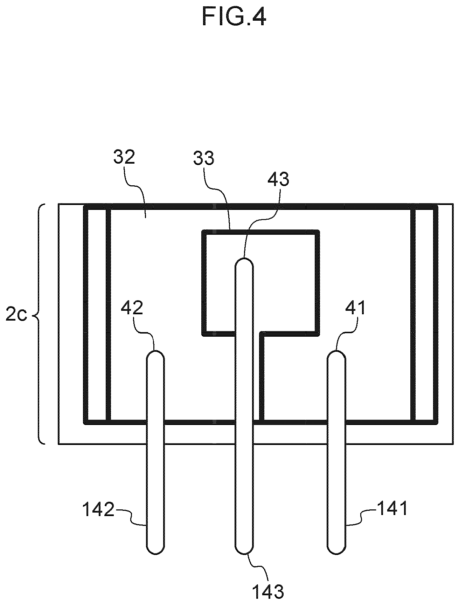

Further, a computing information detection region 2c, which is insulated from the component measurement region 2b by trimming, is provided at the downstream side of the component measurement region 2b. Fig. 3 and Fig. 4 are drawings illustrating the computing information detection region 2c of the biosensor 2 in an enlarged manner. As illustrated in Fig. 3, a conductive region 32, which is formed of a conductive material that is a nickel vanadium alloy that is the same as the electrode layer, is formed at the computing information detection region 2c of the biosensor 2. A rectangular cutting line 33 is formed at the conductive region 32 by trimming by laser light, and the cutting line 33 portion is not conductive. However, the conductive region 32 is not disconnected, and is conductive overall.

Further, as illustrated in Fig. 4, due to the conductive region 32 contacting electrodes provided at the measuring device 1, a voltage application point 41 to which a predetermined voltage is applied, an earth connection point 42 connected to ground potential, and a measurement point 43 for measuring an electrical measurement value, are established. Note that the cutting line 33 that surrounds the periphery of the measurement point 43 is not a complete rectangle, and a portion thereof is open as illustrated in Fig. 3. Due to a portion being open, when voltage is applied to the voltage application point 41, measurement of the electrical measurement value at the measurement point 43 is possible.

A voltage application terminal 141, a ground terminal 142 and a measurement terminal 143, which are provided at the measuring device 1 and contact the conductive region 32 of the computing information detection region 2c due to insertion of the biosensor 2, are illustrated in Fig. 4. The voltage application terminal 141 is a terminal that is connected to the voltage application point 41 in order to apply voltage to the conductive region 32. The ground terminal 142 is a terminal that is connected to the earth connection point 42 in order to make the earth connection point 42 ground potential. The measurement terminal 143 is a terminal that is connected to the measurement point 43 in order to measure the electrical measurement value of the measurement point 43. Note that the electrical measurement value is the potential value, the current value, the resistance value, or another value that can be acquired electrically. There may be one of the measurement point 43 and the measurement terminal 143 respectively, or there may be two or more of each. Further, there may be a structure in which the respective distal end sides of the voltage application terminal 141 and the ground terminal 142 are bifurcated and contact the conductive region 32 at two points. In this case, there are two of each of the voltage application point 41 and the earth connection point 42 as well.

The computing information detection region 2c has a region that prescribes the ratio between the resistance value (first resistance value) at the conductive region 32 between the voltage application point 41 and the earth connection point 42, and the resistance value (second resistance value) at the conductive region 32 between the voltage application point 41 or the earth connection point 42 and the measurement point 43. This region is the resistance prescription region. The cutting line 33 is the resistance prescription region.

When the biosensor 2 is inserted in the measuring device 1 and voltage is applied by the voltage application terminal 141, current flows from the voltage application point 41 toward the earth connection point 42. Further, due to a portion of the cutting line 33 being open (not disconnected) as illustrated in Fig. 3, the measuring device 1 can measure the electrical measurement value at the measurement point 43 that is positioned midway along the path along which current flows from the voltage application point 41 toward the earth connection point 42.

In a case in which the resistance value or the absence/presence of connection between two points at the conductive material is used as an indicator of respective computing information, even if the shapes of conductive materials are the same, the resistance values between two points will differ if there are differences in the manufacturing of the conductive materials themselves, or if there is damage to the conductive materials. Resistance value R can be expressed as

$$R = r \cdot L/t \cdot W$$

r is the resistivity of the conductive material, L is the length between two points of the conductive material, t is the thickness between two points of the conductive material, and W is the width between two points of the conductive material. Here, if there is a difference in the manufacturing of the conductive materials themselves, or if there is damage to the conductive materials, r in the above formula varies, and even if the conductive materials have the same length, thickness and width, the resistance values of these conductive materials will be different.

At the biosensor 2 relating to the present embodiment, due to the voltage application point 41, the earth connection point 42 and the measurement point 43 being established at the conductive region 32, effects of the resistance value of the conductive material itself on the detecting of the computing information at the measuring device 1 can be suppressed. Namely, at the biosensor 2 relating to the present embodiment, the ratio between the resistance value at the conductive region 32 between the voltage application point 41 and the earth connection point 42, and the resistance value at the conductive region 32 between the voltage application point 41 or the earth connection point 42 and the measurement point 43, is prescribed by the cutting line 33. Even if in the above formula varies, detection of the computing information is carried out in accordance with the ratio of the resistance values and not the resistance value of the biosensor 2 relating to the present embodiment. Therefore, in the detecting of the computing information at the measuring device 1, effects of the resistance value of the conductive material itself can be suppressed.

Fig. 5 is a block drawing illustrating the functions of the measuring device 1 of the present embodiment. As described above, the measuring device 1 of the present embodiment is equipped with the biosensor 2 that has the first electrode pair 10 formed from the first working electrode 11 and the first counter electrode 12 and measuring an object of measurement within blood, the second electrode pair 20 formed from the second working electrode 21 and the second counter electrode 22 and formed at the upstream side of the first electrode pair 10, the blood sensing electrode 30 formed at the downstream side of the first electrode pair 10, and the reagent 40 that is placed so as to contact at least the first working electrode 11 and that reacts with the object of measurement.

The lead portions 11a, 12a, 21a, 22a and 30a of the respective electrodes of the biosensor 2 are respectively connected in parallel to ground and to the voltage applying device 50 that is described later, by connecting circuits 200, 300. At the connecting circuit 200, a switch 211 for the first working electrode is provided between the voltage applying device 50 and the lead portion 11a, a switch 212 for the first counter electrode is provided between the voltage applying device 50 and the lead portion 12a, a switch 221 for the second working electrode is provided between the voltage applying device 50 and the lead portion 21a, a switch 222 for the second counter electrode is provided between the voltage applying device 50 and the lead portion 22a, a switch 230 for the blood sensing electrode is provided between the voltage applying device 50 and the lead portion 30a, and a switch 241 for computing information acquisition is provided between the voltage applying device 50 and the voltage application point 41.

Moreover, the paths between the lead portions 11a, 12a, 21a, 22a and 30a of the respective electrodes and the switches 211, 212, 221, 222, 230 corresponding respectively thereto are each bifurcated, and are connected in parallel to ground. A ground switch 311 for the first working electrode, a ground switch 312 for the first counter electrode, a ground switch 321 for the second working electrode, a ground switch 322 for the second counter electrode, and a ground switch 330 for the blood sensing electrode are respectively provided at the connecting circuit 300 that is between ground and these respective points of bifurcation. These respective switches are electron switches, and, as described above, the on/off states thereof are controlled by the control section 100.

Further, the voltage application point 41 of the biosensor 2 is connected by the connecting circuit 200 to the voltage applying device 50 that is described later. The earth connection point 42 is connected to ground. The measurement point 43 is connected to the control section 100.

The measuring device 1 has the voltage applying device 50 that is equipped with a power source. The voltage applying device 50 can be connected to the respective electrodes through the connecting circuit 200. Further, the voltage applying device 50 has a current/voltage converting circuit 51 that converts the current flowing between electrodes into voltage and outputs the voltage, and an A/D converting circuit 52 that converts the voltage value from the current/voltage converting circuit 51 into a pulse. Due to the desired switches corresponding to the electrodes that are to be used being turned on by the control section 100, and the voltage that is applied between the respective electrodes being variably controlled at ground, the voltage applying device 50 applies pulse voltage between electrodes, and can acquire the response current value that flows between the electrodes.

The voltage value of the applied bias voltage is, for example, set in the range of 50 ~ 1000 mV, and is more preferably set in the range of 100 ~ 600 mV. Further, the peak value of the response current is, for example, set to be greater than or equal to 0.3 uA, and is more preferably set to be greater than or equal to 0.7 uA. As an example, the voltage applying device 50 applies voltage of 200 mV or 500 mV to the voltage application point 41, and applies voltage of 400 mV to the lead portion 11a, and applies voltage of 500 mV to the lead portion 30a.

The measuring device 1 further has the control section 100 that is a central control device that executes predetermined programs. The control section 100 controls the opening and closing of the respective switches of the connecting circuits 200, 300, and, on the basis of the pulse from the A/D converting circuit 52 of the voltage applying device 50, acquires measured data expressing a measurement value. The control section 100 also carries out display control with respect to the display screen 1c. In addition to the measured data, information as to whether or not the biosensor 2 is a suitable implement is displayed on the display screen 1c. The control section 100 judges whether or not the biosensor 2 is a suitable implement by specifying the information for computing.

The control section 100 includes, as the functional structures thereof, an information measuring section 101, an information for computing specifying section 102, and a component measuring section 103. The control section 100 functions so as to have the information measuring section 101, the information for computing specifying section 102 and the component measuring section 103, due to a CPU (Central Processing Unit) reading-out and executing a program that is recorded in a ROM (Read Only Memory) or a storage.

The information measuring section 101 measures an electrical measurement value of the measurement point 43. The electrical measurement value of the measurement point 43 is obtained by the biosensor 2 being inserted in the measuring device 1, and the measurement terminal 143 being connected to the measurement point 43. When the information measuring section 101 senses that the biosensor 2 has been inserted in the measuring device 1, the information measuring section 101 effects control so as to connect the switch 241 for computing information acquisition, and effects control so as to set the other switches in non-connected states. Due to the switch 241 for computing information acquisition being connected, a predetermined voltage is applied from the voltage applying device 50 through the voltage application terminal 141 to the voltage application point 41. When the predetermined voltage is applied to the voltage application point 41, a potential difference arises between the voltage application point 41 and the earth connection point 42 that is made to be ground potential by the ground terminal 142, and due thereto, current flows from the voltage application point 41 to the earth connection point 42. Due to current flowing from the voltage application point 41 to the earth connection point 42, the electrical measurement value of the measurement point 43 that is between the voltage application point 41 and the earth connection point 42 passes through the measurement terminal 143 and can be measured at the information measuring section 101 as potential for example.

On the basis of the electrical measurement value measured by the information measuring section 101, the information for computing specifying section 102 specifies the computing information of the biosensor 2 inserted in the measuring device 1. Because the electrical measurement value corresponds to the ratio between the first resistance value and the second resistance value, it can also be said that the computing information is specified on the basis of the ratio between the first resistance value and the second resistance value. In detail, at the time of specifying the information for computing, the information for computing specifying section 102 specifies the computing information of the biosensor 2 by comparing a value that is stored in advance and the electrical measurement value that is measured by the information measuring section 101.

By the terminals 111, 112, 121, 122, 130 for component measurement, the component measuring section 103 measures components contained in the sample on the basis of the electrical measurement values of the respective lead portions 11a, 12a, 21a, 22a, 30a of the biosensor 2. In the present embodiment, the component measuring section 103 measures the glucose value and the hematocrit value within the blood. Further, in the present embodiment, as a result of the computing information being specified by the information for computing specifying section 102, measurement of components by the component measuring section 103 is carried out only in cases in which the biosensor 2 is a sensor that corresponds to the measuring device 1.

In a case of measuring information of the glucose value, the component measuring section 103 effects control so as to connect the switch 211 for the first working electrode and the ground switch 311 for the first working electrode, and effects control so as to set the other switches in non-connected states. Then, when the voltage applying device 50 applies DC voltage (e.g., 400 mV) to the first electrode pair 10, the component measuring section 103 measures the response current value that corresponds to the applied DC voltage. The voltage value of the DC voltage applied to the first electrode pair 10 is, for example, set in the range of 100 ~ 1000 mV, and is more preferably set in the range of 200 ~ 500 mV. The component measuring section 103 may use the measured response current value as is as the information of the glucose value. Or, the component measuring section 103 may use, as the glucose value, a value that has been converted into the concentration of the object of measurement, by referring to a calibration line or a comparison table that has been prepared by current response values measured at objects of measurement of concentrations that are known in advance.

In a case of measuring information of the hematocrit value, the component measuring section 103 effects control so as to connect the switch 221 for the second working electrode and the ground switch 321 for the second working electrode, and effects control so as to set the other switches in non-connected states. Then, when the voltage applying device 50 applies DC voltage (e.g., 4000 mV = 4 V) to the second electrode pair 20, the component measuring section 103 measures the response current value corresponding to the applied DC voltage. The voltage value of the DC voltage applied to the second electrode pair 20 is, for example, set in the range of 2 ~ 20 V, and is more preferably set in the range of 3 ~ 10 V. The component measuring section 103 may use the measured response current value as is as the information of the hematocrit value. Or, the component measuring section 103 may use, as the hematocrit value, a value that has been converted into the concentration of the object of measurement, by referring to a calibration line or a comparison table that has been prepared by current response values measured at other blood samples that are known in advance.

In the measuring device of the above-described embodiment, at the time when pulse voltage is applied to the respective electrodes, the control section 100 carries out control of opening/closing the respective switches. However, in the measuring device 1 of another embodiment, pulse voltage may be applied directly from the voltage applying device 50 to the respective electrodes on the basis of control from the control section 100, without providing the respective switches.

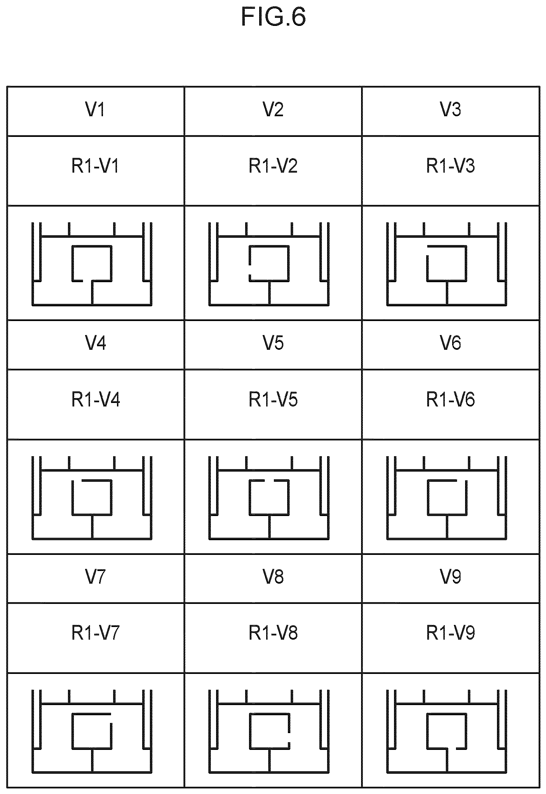

The shape of the conductive region 32 is not limited to that illustrated in Fig. 3 and Fig. 4. Fig. 6 is a drawing illustrating modified examples of the shape of the conductive region 32. In V1, the open position is closest to the earth connection point 42, and the open positions become further away from the earth connection point 42 in the order of V2, V3, ..., and the open position is furthest from the earth connection point 42 in V9. In other words, in V9, the open position is closest to the voltage application point 41. As illustrated in Fig. 6, the electrical measurement value of the measurement point 43 can be varied by selecting the open position at the cutting line 33 from among various patterns.

Table 1 is a table listing measured potentials of the measurement point 43 at the time when voltage of 200 mV is applied to the voltage application point 41. The measurement points 43 are all the same point. As illustrated in Table 1, the measured potential of the measurement point 43 can be adjusted by changing the shape of the conductive region 32, i.e.,

the open position at the cutting line 33.

[Table 1]

(Table 1: Measured Potential (mV) When Potential of 200 mV is Applied)

|  | V1 | V2 | V3 | V4 | V5 | V6 | V7 | V8 | V9 |
|---|---|---|---|---|---|---|---|---|---|
| average | 201.0 | 185.5 | 166.0 | 144.0 | 102.4 | 68.1 | 40.3 | 22.4 | 5.5 |
| average + 3SD | 200.2 | 179.9 | 162.3 | 134.0 | 99.6 | 64.0 | 35.3 | 18.2 | 3.2 |
| average - 3SD | 201.8 | 191.2 | 169.7 | 146.0 | 105.1 | 72.1 | 45.2 | 26.6 | 7.8 |

Because the electrical measurement value can be selected from among nine electrical measurement values in the form illustrated in Fig. 6, nine patterns can be readied as indicators. Specifically, plural calibration curves can be readied such that there is calibration curve No. 1 at a measured potential of 200 V $\pm$ 5V, and calibration curve No. 2 at a measured potential of 185 V $\pm$ 5V, ..., and calibration curve No. 9 at a measured potential of 5 V $\pm$ 5V, and it can be said that, at the time of outputting the information for computing of this process, it is difficult to be affected by the resistance value of the conductive material itself. At the computing information detection region 2c, the shape (surface area and contour) of the conductive region 32 that includes the voltage application point 41, the earth connection point 42 and the measurement point 43 is the same, and only the shape of the cutting line 33, i.e., the portion that is open, differs. Therefore, there is also the advantage that manufacturing is easy.

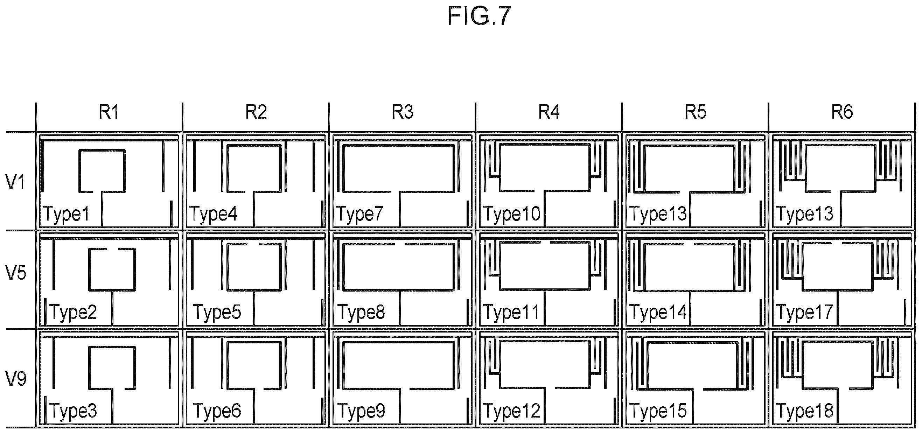

Moreover, the electrical measurement value of the measurement point 43 can also be varied by changing the shape of the cutting line 33 into different patterns. Fig. 7 is a drawing illustrating modified examples of the shape of the cutting line 33, and shows three types of shapes of the cutting line 33 at the periphery of the measurement point 43, and six types of shapes of the cutting line 33 between the voltage application point 41 and the earth connection point 42, for a total of 18 types of shapes.

The electrical measurement value of the measurement point 43 can be changed by a method other than a method of changing the shape of the cutting line 33. For example, the electrical measurement value of the measurement point 43 can be changed also by trimming a portion of the conductive region 32, or changing the region where the conductive region 32 is sputtered.

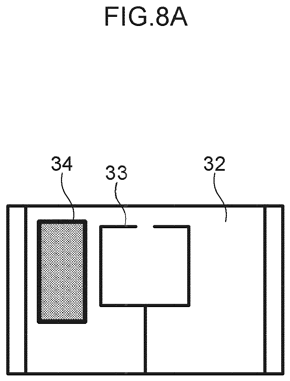

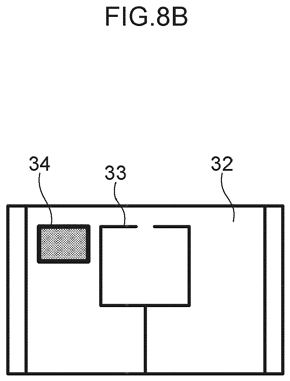

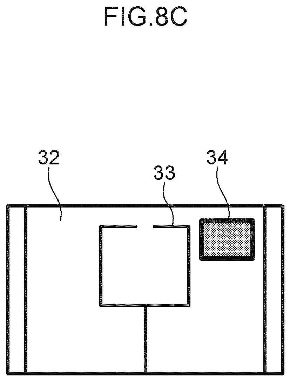

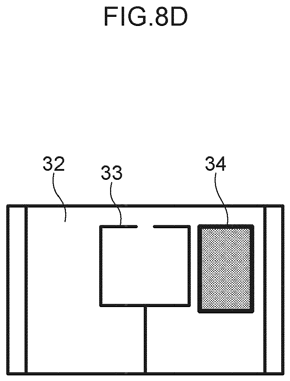

By prescribing the surface area between the voltage application point 41 or the earth connection point 42 and the measurement point 43 by providing a non-conductive region between the voltage application point 41 and the earth connection point 42, as a result, the length and width between the voltage application point 41 and the earth connection point 42, and the length and width between the voltage application point 41 or the earth connection point 42 and the measurement point 43, can be varied. Due thereto, the resistance value between the voltage application point 41 and the earth connection point 42, and the resistance value between the voltage application point 41 or the earth connection point 42 and the measurement point 43, may be prescribed. Fig. 8A through Fig. 8D are drawings illustrating examples of trimming a portion of the conductive region 32. As illustrated in Fig. 8A through Fig. 8D, by trimming a portion of the conductive region 32 at a trimming region 34, the electrical measurement value of the measurement point 43 can be changed. Although the shapes of all of the cutting lines 33 are the same in Fig. 8A through Fig. 8D, the electrical measurement value of the measurement point 43 can be changed by changing the shape of the cutting line 33 after trimming the conductive region 32 at the trimming region 34. Note that the shapes of and the numbers of the trimming regions 34 are not limited to those illustrated in Fig. 8, provided that they do not interfere with the voltage application point 41, the earth connection point 42 and the measurement point 43.

Note that the positions of the voltage application point 41, the earth connection point 42 and the measurement point 43 are not limited to the positions illustrated in Fig. 4. For example, by changing the position of the earth connection point 42, the potential that is measured at the measurement point 43 changes even if the other conditions are the same.

As described above, in accordance with embodiments of the present disclosure, there can be provided the biosensor 2 that, at the time of identifying computing information at the measuring device 1, is not affected by the resistance value of a conductive material itself. Further, in accordance with embodiments of the present disclosure, there can be provided a system comprising the testing implement 2 and the measuring device 1 that, by measuring computing information from the conductive region 32 at at least three points, is not affected by the resistance value of the conductive material itself of the biosensor 2, and can identify the computing information.

**Claims**

1. A testing implement (2) configured to be utilized in a state of attachment to a measuring device (1) for performing computing using information values representing a measurement target component within a sample based on information for computing, the testing implement (2) being configured to output the information for computing to the measuring device (2), the testing implement (1) comprising:

   a component measurement region (2b) disposed on a substrate formed from a non-conductive material, for measuring the measurement target component;
   a conductive region (32) insulated from the component measurement region (2b) formed of a conductive material and disposed on the substrate, and at which a voltage application point (41) to which a predetermined voltage is applicable, an earth connection point (42) for connection to ground potential, and at least one measurement point (43) for measuring an electrical measurement value, are established; and
   a resistance prescription region that prescribes a ratio, within the conductive region (32), between a first resistance value at the conductive region between the voltage application point (41) and the earth connection point (42), and a second resistance value at the conductive region between the voltage application point (41) or the earth connection point (42), and the measurement point (43);
   wherein the resistance prescription region is a region within the conductive region (32);
   wherein the resistance prescription region is a non-conductive region between the voltage point (41) and the earth connection point (42);
   wherein the resistance prescription region is a non-conductive cutting line (33) that incompletely surrounds a periphery of the measurement point (43) .

2. The testing implement (2) of claim 1, wherein the resistance prescription region prescribes the ratio between the first resistance value and the second resistance value by prescribing a distance between the voltage application point (41) and the earth connection point (42), and a distance between the voltage application point (41) or the earth connection point (42), and the measurement point (43).

3. The testing implement (2) of claim 1 or 2, wherein the cutting line (33) surrounds the periphery of the measurement point (43) in a rectangular shape.

4. The testing implement (2) of claim 1 or 2, wherein the cutting line (33) is incomplete at a position in a vicinity of the measurement point (43).

5. The testing implement (2) of claim 1, wherein the resistance prescription region prescribes the ratio between the first resistance value and the second resistance value by prescribing a length and a width between the voltage application point (41) and the earth connection point (42), and a length and a width between the voltage application point (41) or the earth connection point (42), and the measurement point (43).

6. A system comprising a measuring device (1) and the testing implement (2) of one of claims 1 to 5 attached thereto, the measuring device (1) for carrying out computation using information values representing a measurement target component within a sample based on the information for computing output by the testing implement (2), the measuring device comprising:

   a voltage application terminal (141) for connection to the voltage application point (41) in order to apply voltage to the conductive material;
   a ground terminal (142) for connection to the earth connection point (42) in order to configure the earth connection point at ground potential;
   a measurement terminal (143) for connection to the measurement point (43);
   a memory; and
   a processor coupled to the memory, wherein the processor is configured to:

      measure an electrical measurement value of the measurement point (43), and
      specify the information for computing, based on the electrical measurement value that has been measured.

7. The system of claim 6, wherein the electrical measurement value of the measurement point (43) is a potential of the measurement point (43).

8. The system of claim 6, the measurement device (1) further comprising a terminal for component measurement that is connected to the component measurement region, wherein the processor is configured for measuring the measurement target component at the component measurement region.

**Patentansprüche**

1. Testgerät (2), das so konfiguriert ist, dass es in einem Zustand der Anbringung an eine Messvorrichtung (1) verwendet werden kann, um Berechnung unter Verwendung von Informationswerten, die eine Messzielkomponente innerhalb einer Probe repräsentieren, basierend auf Berechnungsinformation durchzuführen, wobei das Testgerät (2) so konfiguriert ist, dass es die Berechnungsinformation an die Messvorrichtung (2) ausgibt; wobei das Testgerät (1) Folgendes umfasst:

   einen Komponentenmessbereich (2b), der auf einem aus einem nichtleitenden Material gebildeten Substrat angeordnet ist, zum Messen der Messzielkomponente;
   ein leitfähiges Material, das auf dem Substrat angeordnet ist und an dem ein Spannungsanlegepunkt (41), an den eine vorbestimmte Spannung anlegbar ist, ein Masseanschlusspunkt (42) zur Verbindung mit Massepotential und mindestens ein Messpunkt (43) zum Messen eines elektrischen Messwerts hergestellt sind; und
   einen Widerstandsvorgabebereich, der innerhalb des leitfähigen Bereichs (32) ein Verhältnis zwischen einem ersten Widerstandswert am leitfähigen Bereich zwischen dem Spannungsanlegepunkt (41) und dem Masseanschlusspunkt (42) und einem zweiten Widerstandswert am leitfähigen Bereich zwischen dem Spannungsanlegepunkt (41) oder dem Masseanschlusspunkt (42) und dem Messpunkt (43) vorgibt;
   wobei der Widerstandsvorgabebereich ein Bereich innerhalb des leitfähigen Bereichs (32) ist;
   wobei der Widerstandsvorgabebereich ein nichtleitender Bereich zwischen dem Spannungspunkt (41) und dem Masseanschlusspunkt (42) ist;
   wobei der Widerstandsvorgabebereich eine nichtleitende Schnittlinie (33) ist, die einen Umfang des Messpunkts (43) unvollständig umgibt.

2. Testgerät (2) nach Anspruch 1, wobei der Widerstandsvorgabebereich das Verhältnis zwischen dem ersten Widerstandswert und dem zweiten Widerstandswert vorgibt, indem er einen Abstand zwischen dem Spannungsanlegepunkt (41) und dem Masseanschlusspunkt (42) und einen Abstand zwischen dem Spannungsanlegepunkt (41) oder dem Masseanschlusspunkt (42) und dem Messpunkt (43) vorgibt.

3. Testgerät (2) nach Anspruch 1 oder 2, wobei die Schnittlinie (33) den Umfang des Messpunkts (43) in einer rechteckigen Form umgibt.

4. Testgerät (2) nach Anspruch 1 oder 2, wobei die Schnittlinie (33) an einer Position in der Nähe des Messpunkts (43) unvollständig ist.

5. Testgerät (2) nach Anspruch 1, wobei der Widerstandsvorgabebereich das Verhältnis zwischen dem ersten Widerstandswert und dem zweiten Widerstandswert vorgibt, indem er eine Länge und eine Breite zwischen dem Spannungsanlegepunkt (41) und dem Masseanschlusspunkt (42) und eine Länge und eine Breite zwischen dem Spannungsanlegepunkt (41) oder dem Masseanschlusspunkt (42) und dem Messpunkt (43) vorgibt.

6. System, umfassend eine Messvorrichtung (1) und das daran angebrachte Testgerät (2) nach einem der Ansprüche 1 bis 5, die Messvorrichtung (1) zum Durchführen von Berechnung unter Verwendung von Informationswerten, die eine Messzielkomponente innerhalb einer Probe repräsentieren, basierend auf der vom Testgerät (2) ausgegebenen Berechnungsinformation, wobei die Messvorrichtung Folgendes umfasst:

   einen Spannungsanlegeanschluss (141) zur Verbindung mit dem Spannungsanlegepunkt (41), um Spannung an das leitfähige Material anzulegen;
   einen Masseanschluss (142) zur Verbindung mit dem Masseanschlusspunkt (42), um den Masseanschlusspunkt auf Massepotential zu konfigurieren;
   einen Messanschluss (143) zur Verbindung mit dem Messpunkt (43);
   einen Speicher; und
   einen Prozessor, der mit dem Speicher gekoppelt ist, wobei der Prozessor konfiguriert ist zum:

      Messen eines elektrischen Messwerts des Messpunkts (43), und

Spezifizieren der Berechnungsinformation basierend auf dem elektrischen Messwert, der gemessen worden ist.

**7.** System nach Anspruch 6, wobei der elektrische Messwert des Messpunkts (43) ein Potential des Messpunkts (43) ist.

**8.** System nach Anspruch 6, wobei die Messvorrichtung (1) weiter einen Komponentenmessanschluss umfasst, der mit dem Komponentenmessbereich verbunden ist, wobei der Prozessor so konfiguriert ist, dass er die Messzielkomponente am Komponentenmessbereich misst.

**Revendications**

**1.** Outil de test (2) configuré pour être utilisé dans un état de fixation à un dispositif de mesure (1) pour effectuer un calcul en utilisant des valeurs d'information représentant un composant cible de mesure dans un échantillon sur la base d'une information de calcul, l'outil de test (2) étant configuré pour délivrer en sortie l'information de calcul au dispositif de mesure (1), l'outil de test (2) comprenant :

une région de mesure de composant (2b) disposée sur un substrat formé à partir d'un matériau non conducteur, pour mesurer le composant cible de mesure ;
un matériau conducteur et disposé sur le substrat, et au niveau duquel un point d'application de tension (41) auquel une tension prédéterminée est applicable, un point de connexion à la terre (42) pour liaison au potentiel de terre, et au moins un point de mesure (43) pour mesurer une valeur de mesure électrique, sont établis ; et
une région de prescription de résistance qui prescrit un rapport, à l'intérieur de la région conductrice (32), entre une première valeur de résistance au niveau de la région conductrice entre le point d'application de tension (41) et le point de connexion à la terre (42), et une deuxième valeur de résistance au niveau de la région conductrice entre le point d'application de tension (41) ou le point de connexion à la terre (42), et le point de mesure (43) ;
dans lequel la région de prescription de résistance est une région à l'intérieur de la région conductrice (32) ;
dans lequel la région de prescription de résistance est une région non conductrice entre le point d'application de tension (41) et le point de connexion à la terre (42) ;
dans lequel la région de prescription de résistance est une ligne de découpe non conductrice (33) qui entoure de manière incomplète une périphérie du point de mesure (43).

**2.** Outil de test (2) selon la revendication 1, dans lequel la région de prescription de résistance prescrit le rapport entre la première valeur de résistance et la deuxième valeur de résistance en prescrivant une distance entre le point d'application de tension (41) et le point de connexion à la terre (42), et une distance entre le point d'application de tension (41) ou le point de connexion à la terre (42), et le point de mesure (43).

**3.** Outil de test (2) selon la revendication 1 ou 2, dans lequel la ligne de découpe (33) entoure la périphérie du point de mesure (43) sous une forme rectangulaire.

**4.** Outil de test (2) selon la revendication 1 ou 2, dans lequel la ligne de découpe (33) est incomplète au niveau d'une position dans un voisinage du point de mesure (43).

**5.** Outil de test (2) selon la revendication 1, dans lequel la région de prescription de résistance prescrit le rapport entre la première valeur de résistance et la deuxième valeur de résistance en prescrivant une longueur et une largeur entre le point d'application de tension (41) et le point de connexion à la terre (42), et une longueur et une largeur entre le point d'application de tension (41) ou le point de connexion à la terre (42), et le point de mesure (43).

**6.** Système comprenant un dispositif de mesure (1) et l'outil de test (2) de l'une des revendications 1 à 5 qui y est fixé, le dispositif de mesure (1) pour effectuer un calcul en utilisant des valeurs d'information représentant un composant cible de mesure dans un échantillon sur la base de l'information de calcul délivrée en sortie par l'outil de test (2), le dispositif de mesure comprenant :

une borne d'application de tension (141) pour liaison au point d'application de tension (41) afin d'appliquer une tension au matériau conducteur ;
une borne de terre (142) pour liaison au point de connexion à la terre (42) afin de configurer le point de connexion à la terre au potentiel de terre ;
une borne de mesure (143) pour liaison au point de mesure (43) ;

une mémoire ; et

un processeur couplé à la mémoire, dans lequel le processeur est configuré pour : mesurer une valeur de mesure électrique du point de mesure (43), et spécifier l'information de calcul, sur la base de la valeur de mesure électrique qui a été mesurée.

7. Système selon la revendication 6, dans lequel la valeur de mesure électrique du point de mesure (43) est un potentiel du point de mesure (43).

8. Système selon la revendication 6, le dispositif de mesure (1) comprenant en outre une borne de mesure de composant qui est reliée à la région de mesure de composant, dans lequel le processeur est configuré pour mesurer le composant cible de mesure au niveau de la région de mesure de composant.

# FIG.1

# FIG.2

FIG.3

# FIG.4

FIG.5

CURRENT/VOLTAGE
CONVERTING CIRCUIT

A/D
CONVERTING CIRCUIT

INFORMATION
MEASURING SECTION

INFORMATION FOR
COMPUTING
SPECIFYING SECTION

COMPONENT
MEASURING SECTION

DISPLAY
SCREEN

EP 4 239 328 B1

# FIG.6

| V1 | V2 | V3 |
|---|---|---|
| R1-V1 | R1-V2 | R1-V3 |
| | | |
| V4 | V5 | V6 |
| R1-V4 | R1-V5 | R1-V6 |
| | | |
| V7 | V8 | V9 |
| R1-V7 | R1-V8 | R1-V9 |
| | | |

# FIG.7

|  | R1 | R2 | R3 | R4 | R5 | R6 |
|---|---|---|---|---|---|---|
| V1 | Type1 | Type4 | Type7 | Type10 | Type13 | Type13 |
| V5 | Type2 | Type5 | Type8 | Type11 | Type14 | Type17 |
| V9 | Type3 | Type6 | Type9 | Type12 | Type15 | Type18 |

EP 4 239 328 B1

FIG.8A

FIG.8B

34    33    32

FIG.8C

FIG.8D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4885508 B
- JP 2003029804 A
- JP 3859239 B
- US 2003029804 A
- WO 2017039976 A
- WO 2009031761 A
- US 20090030617 A1